Europäisches Patentamt

European Patent Office

Office européen des brevets

(19)

(11) EP 0 632 886 B1

(12) EUROPÄISCHE PATENTSCHRIFT

(45) Veröffentlichungstag und Bekanntmachung des Hinweises auf die Patenterteilung:
**22.09.1999 Patentblatt 1999/38**

(21) Anmeldenummer: 93906497.8

(22) Anmeldetag: **10.03.1993**

(51) Int Cl.6: **G01N 3/20**, G01N 33/46

(86) Internationale Anmeldenummer:
**PCT/EP93/00547**

(87) Internationale Veröffentlichungsnummer:
**WO 93/19355 (30.09.1993 Gazette 1993/24)**

(54) **VERFAHREN UND VORRICHTUNG ZUR MASCHINELLEN FESTIGKEITSSORTIERUNG VON SCHNITTHOLZ**

PROCESS AND DEVICE FOR MACHINE SORTING OF SAWN TIMBER ACCORDING TO STRENGTH

PROCEDE ET DISPOSITIF DE TRIAGE MECANIQUE DE BOIS SCIE EN FONCTION DE LA RESISTANCE

(84) Benannte Vertragsstaaten:
**AT CH DE DK FR GB IT LI NL SE**

(30) Priorität: **23.03.1992 DE 4209314**

(43) Veröffentlichungstag der Anmeldung:
**11.01.1995 Patentblatt 1995/02**

(73) Patentinhaber: **FAGUS-GRECON GRETEN GMBH & CO. KG**
**31061 Alfeld (DE)**

(72) Erfinder:
- **PALM, Klaus**
  **D-3223 Grünenplan (DE)**
- **WIENCKOWSKI, Detlef**
  **D-3256 Coppenbrügge 8 (DE)**
- **STEINBACH, Martin**
  **D-3212 Gronau (Leine) (DE)**

(74) Vertreter: **Kosel, Peter, Dipl.-Ing. et al**
**Patentanwälte**
**Kosel & Sobisch**
**Odastrasse 4a**
**37581 Bad Gandersheim (DE)**

(56) Entgegenhaltungen:
**GB-A- 1 104 986       GB-A- 2 105 856**

- **MICROTECNIC Nr. 1, 1991, ZURICH CH Seiten 10 - 13 HOFFMANN 'PSD-Elemente erweitern die optische Messtechnik'**
- **HOLZ-ZENTRALBLATT Nr. 97, 14. August 1987, STUTTGART,GERMANY Seite 1360 TEBBE 'Zum gegenwärtigen Stand der maschinellen Holzsortierung'**
- **JOURNAL OF VACUUM SCIENCE AND TECHNOLOGY: PART A Bd. 9, Nr. 4, 1991, NEW YORK US Seiten 2204 - 2209 CARDINALE ET AL. 'biaxial modulus measurement ...'**

**Beschreibung**

Die Erfindung betrifft ein Verfahren nach dem Oberbegriff des Anspruchs 1.

Bei einem bekannten Verfahren dieser Art (GB 2 105 856 A) wird die Meßrolle 9 durch ein Handrad 15 in Abhängigkeit von der Art des zu sortierenden Schnittholzes L in eine dann nicht mehr veränderte Betriebsstellung eingestellt. Die Achse der Meßrolle 9 ist an einem stabilen Arm 13 gelagert, der seinerseits an einer Achse 14 des Maschinenrahmens 10 schwenkbar gelagert ist. Die natürliche Vorkrümmung des Holzes wird nicht gemessen. Stattdessen wird jedes Holz nach dem ersten Durchlauf um 180° um seine Längsachse gewendet, in entgegengesetzter Richtung durch die Maschine hindurch zum Einlaß der Maschine zurücktransportiert und in einem zweiten Durchlauf erneut durch die Maschine geschickt. Dies ist zeit- und arbeitsaufwendig und kann bei bestehender natürlicher Vorkrümmung zu einer Überbeanspruchung des Holzes führen. Auch müssen die Drehantriebe der getriebenen Klemmrollen 2, 4 umsteuerbar sein.

Aus der GB 1 104 986 ist ein Verfanren an sich bekannt, bei dem nur ein Paar drehend antreibbarer Rollen 2, 4 vorgesehen ist, von denen eine 4 quer vorspannbar 5 ist. Auf der Seite der stationären Rolle 2 und im Abstand davon befindet sich eine stationäre Stützrolle 3. Auf der Seite der vorspannbaren Rolle 4 ist zwischen dem Rollenpaar 2, 4 und der Stützrolle 3 eine Meßrolle 7 durch einen Antrieb 8 mit konstanter (Seite 2, Zeile 115) Kraft an das Schnittholz andrückbar. Die sich dadurch ergebende effektive Durchbiegung des Holzes wird durch ein an der gegenüberliegenden Seite des Holzes anliegendes Tastelement 9 abgetastet und über Mikroschalter 11 in entsprechende elektrische Signale gewandelt, die in eine Auswerteschaltung eingegeben werden. Die effektive Durchbiegung kann durch eine natürliche Vorkrümmung des Holzes verfälscht sein. Deshalb ist vor dem Rollenpaar 2, 4 ein Meßarm 15 angeordnet, der um die Achse der stationären Rolle 2 schwenkbar ist. Im gleichen Abstand, wie ihn die Rollen 2 und 3 voneinander aufweisen, ist vor der Rolle 2 eine Rolle 13 stationär an dem Meßarm 15 angeordnet. Die Rolle 13 wird durch eine vorgespannte 18 Gegenrolle 14 in ständiger Anlage am Holz gehalten. In der gleichen geometrischen Position wie das Tastelement 9 relativ zu den Rollen 2, 3 ist ein durch einen Arm 16 des Meßarms 15 gehaltenes Tastelement zum Tasten der natürlichen Vorkrümmung des Holzes relativ zu den Rollen 2, 13 angeordnet. Die Schwenkungen des Armes 16 relativ zu dem Meßarm 15 werden in elektrische Signale gewandelt 19, die der natürlichen Vorkrümmung entsprechen. In der Auswerteschaltung erfolgt die Verrechnung der natürlichen Vorkrümmung mit der an dem Tastsystem 9 bis 11 gemessenen effektiven Durchbiegung des Holzes. Die konstante Andrückkraft der Meßrolle 7 berücksichtigt nicht einen sich ändernden Elastizitätsmodul der Hölzer.

Aus der Zeitschrift "Microtecnic" Nr. 1, 1991, Zürich, CH, Seite 10 bis 13, Hofmann "PSD-Elemente erweitern die optische Meßtechnik", sind verschiedene optische Distanzsensoren zur berührungslosen Distanzmessung an sich bekannt.

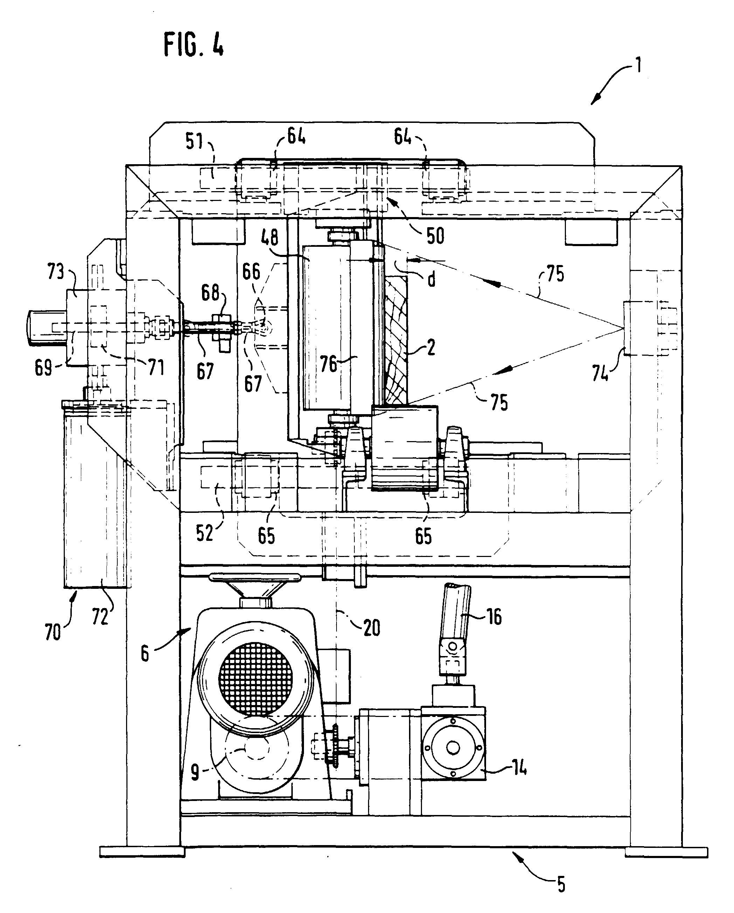

Aus der Zeitschrift "Holz-Zentralblatt" Nr. 97, 14. August 1987, Stuttgart, Deutschland, Seite 1360, Tebbe "Zum gegenwärtigen Stand der maschinellen Gütesortierung", Spalte 3, Zeile 31 bis 53; Abbildung 4, ist es an sich bekannt, eine entscheidende Verbesserung der Sortierung aus der Kombination von Sortierparametern zu erwarten. Möglich sei die Kombination von Elastizitätsmodul und Ästigkeit. Die Ästigkeit könne z. B. mit einem Durchstrahlungsverfahren (Mikrowellen oder Röntgenstrahlen) bestimmt werden.

Der Erfindung liegt die Aufgabe zugrunde, bei nur einem Durchlauf des Holzes den Durchsatz und die Qualität der Festigkeitssortierung zu verbessern.

Diese Aufgabe ist verfahrensmäßig durch die Merkmale des Anspruchs 1 gelöst. Der Durchsatz ist wegen des nur einen erforderlichen Durchlaufs jedes Holzes gesteigert. Wegen der zumindest annähernd konstant gehaltenen erzwungenen Durchbiegung hält sich die Beanspruchung des Holzes in definierbaren Grenzen. Besonders vorteilhaft ist dabei, daß eine eventuelle natürliche Vorkrümmung des Holzes vorab erfaßt sowie rechnerisch und durch dem Rechenergebnis entsprechende Regelung der Stellung der Meßrolle quer zu der Bewegungsrichtung des Holzes kompensiert werden kann. Die Messung dieser Stellung der Meßrolle liefert dem Rechner stets aktuelle Werte der Ist-Stellung der Meßrolle.

Die Merkmale des Anspruchs 2 sorgen für eine schonende Behandlung des Holzes.

Gemäß Anspruch 3 sind die Ruckschlüsse entsprechend sicher, die man aus der Messung der Rückstellkraft auf die Festigkeit des Holzes zieht.

Gemäß Anspruch 4 ergibt sich der besondere Vorteil, daß die Vorderkante des Holzes beim Einlauf in ein Klemmrollenpaar nicht unnötig belastet oder gar beschädigt wird.

Die Merkmale des Anspruchs 5 dienen der schnellen Vorbereitung auf das nächste Holz.

Die Messungen gemäß Anspruch 6 und 7 werden vorzugsweise berührungslos erfolgen und können laufend oder periodisch durchgeführt werden.

Gemäß Anspruch 8 ergibt sich eine sehr zuverlässige Ermittlung des örtlichen Biege-Elastizitätsmoduls.

Die Merkmale des Anspruchs 9 bringen den Vorteil, daß auf diese Weise auch die Festigkeit an Anfang und Ende des Holzes untersucht werden können, die bei einem reinen Biegeverfahren wegen der dort unerläßlichen Stützweite nicht geprüft werden könnten. Die Bewertung der Festigkeitsmeßwerte an Anfang und Ende des Holzes kann alle dafür gegebenenfalls bestehenden besonderen Festigkeitsbedingungen berücksichtigen. Z.B. bei Hölzern, die für Keilzinkung oder Nagelbinder und anderes Konstruktionsholz mit Anschluß mechanischer Verbindungselemente vorgesehen sind, müssen Anfang und Ende frei von Ästen sein und dürfen eine bestimmte Rohdichte nicht unterschreiten.

Gemäß Anspruch 10 steht eine besonders sichere Durchstrahlungsquelle zur Verfügung, die außerdem getaktet betrieben, also dann ausgeschaltet werden kann, wenn Meßdaten aus einer Durchstrahlung nicht erforderlich sind. Ein Röntgenstrahler läßt sich verhältnismäßig leicht betriebssicher abschirmen und handhaben.

Durch die Zusammenfassung der Kennwerte gemäß Anspruch 11 kann die Sicherheit und Präzision der Festigkeitsbeurteilung des Holzes erheblich gesteigert werden. Dies führt zu einer besseren Ausnutzung des Naturstoffes Holz und kann andererseits zu Verringerung der Holzquerschnitte bei gleicher Festigkeit führen. Der Bewertung der einzelnen Meßwerttypen im Rahmen ihrer rechnerischen Zusammenfassung zur Festlegung einer Sortierklasse sind keine Grenzen gesetzt. So kann z.B. im Anfangs- und Endbereich des Holzes im Sinne des zu Anspruch 9 Gesagten besondere Betonung auf Ästigkeit und Rohdichte gelegt werden, während im sonstigen Bereich des Holzes der Biegefestigkeit besonderes Gewicht beigemessen wird.

Ein Mittel dazu kennzeichnet Anspruch 12. Damit ist den beteiligten Verkehrskreisen eine verläßliche Auswahl von Hölzern in den einzelnen Sortierklassen ermöglicht. In an sich üblicher Weise kann außerdem eine Markierung des Holzes mit diesen Sortierklassen erfolgen.

Die Merkmale des Anspruchs 13 gestatten eine noch bessere Ausbeute aus vorhandenem Holz. So lassen sich höchstmögliche Anteile des Holzes jeweils in die höchstmöglichen Sortierklassen einstufen. Auch hier erfolgt zweckmäßigerweise eine Markierung mit der jeweiligen Sortierklasse, um das automatisierte weitere Handling des Holzes zu erleichtern.

Die Erfindung betrifft auch eine Vorrichtung nach dem Oberbegriff des Anspruchs 14.

Eine solche Vorrichtung ist durch die vorerwähnte GB 2 105 856 A bekannt. Diese Maschine besitzt die zuvor auf der Verfahrensseite angemerkten Nachteile.

Der Erfindung liegt auch die Aufgabe zugrunde, die Vorrichtung zu verbessern.

Diese Aufgabe ist durch die Merkmale des Anspruchs 14 gelöst. Für die Vorrichtung ergeben sich im wesentlichen die gleichen Vorteile wie zuvor zu Anspruch 1 geltend gemacht.

Gemäß Anspruch 15 sind die Längsachsen der Klemmrollen jedes Klemmrollenpaars vorzugsweise parallel zueinander angeordnet. Die Aufnahme der zweiten Klemmrolle in einem Schlitten oder alternativ einem Wagen gewährleistet eine besonders präzise und reibungsarme Querbewegung der zweiten Klemmrolle. Als Klemmantrieb kann z.B. ein durch den Rechner steuerbarer, mit Druckluft betätigbarer Balgzylinder verwendet werden. Solche Balgzylinder werden z.B. von der Firma Robert Bosch GmbH in Stuttgart, Deutschland, vertrieben.

Die Ausbildung gemäß Anspruch 16 verkürzt die durch den Klemmantrieb zu überwindenden Wege auf ein Minimum. Die Voreinstellung des Halters kann z.B. von Hand durch einen Spindeltrieb erfolgen.

Die Merkmale des Anspruchs 17 schaffen eine definierte Ausgangsstellung für den Schlitten.

Die berührungslose Messung gemäß Anspruch 18 ist verschleißfrei und sehr präzise. Als Meßelemente können z.B. "MQ" Laser Analog Sensoren verwendet werden, die durch die SDS-RELAIS AG, Fichtenstraße 3-5, D-82041 Deisenhofen bei München, geliefert werden.

Die Dickenmessung gemäß Anspruch 19 erfolgt vorzugsweise vor dem zuerst mit dem Holz in Berührung tretenden Klemmrollenpaar und vorzugsweise ebenfalls mit den vorerwähnten Lasersensoren.

Gemäß Anspruch 20 ist die Möglichkeit geschaffen, zusätzlich zu dem Biege-Elastizitätsmodul aufgrund der Durchbiegung des Holzes auch Meßdaten zur Ästigkeit und/oder Rohdichte in die Abschätzung der Festigkeit des Holzes einzubeziehen.

Der Röntgenstrahler gemäß Anspruch 21 und die zugehörige Empfängerzeile liefern über die gesamte Breite des Holzes Bildpunkte, die bei Zusammenschau mit den benachbarten Bildzeilen eine sehr sichere und vollständige Bildauswertung ermöglichen. So läßt sich die Lage von Fehlstellen wie Ästen und sonstigen Rohdichteschwankungen auch in Querrichtung des Holzes genau feststellen, beurteilen und bewerten.

Besonders zweckmäßig sind dabei die Merkmale des Anspruchs 22.

Gemäß Anspruch 23 ist die Meßrolle besonders präzise und reibungsarm in Querrichtung verschiebbar. Entsprechend sicher ist die Ermittlung der Rückstellkraft durch die Kraftmeßvorrichtung. Anstelle eines Schlittens kann auch hier ein Wagen verwendet werden.

Gemäß Anspruch 24 wird eine einwandfreie Kraftmessung auch dann gewährleistet, wenn die durch das Holz auf die Meßrolle ausgeübte resultierende Kraft nicht in der Längsmitte der Meßrolle angreift.

Die Merkmale des Anspruchs 25 kennzeichnen einen besonders schnellen und sehr genauen Stellantrieb.

Gemäß Anspruch 26 ergeben sich sehr genaue Wegsignale.

Gemäß Anspruch 27 läßt sich das Holz auf einfache Weise für vielfältige nachfolgende Auswertung markieren.

Weitere Merkmale und Vorteile der Erfindung werden nachfolgend anhand der in den Zeichnungen dargestellten Ausführungsbeispiele näher erläutert. Es zeigt:

Fig. 1 eine Seitenansicht einer Vorrichtung zur maschinellen Festigkeitssortierung von Schnittholz,

Fig. 2 die Draufsicht auf die Vorrichtung gemäß Fig. 1,

Fig. 3 die Ansicht auf die Vorrichtung von links in Fig. 1,

Fig. 4 im wesentlichen die Schnittansicht nach Linie IV-IV in Fig. 1,

Fig. 5 eine Einzelheit aus Fig. 1 in vergrößerter Darstellung,

Fig. 6 die Draufsicht auf die Einzelheit gemäß Fig. 5,

Fig. 7 die Ansicht VII in Fig. 5,

Fig. 8 eine Einzelheit aus Fig. 1 in vergrößerter Darstellung,

Fig. 9 die Draufsicht auf die Einzelheit gemäß Fig. 8,

Fig. 10 die Ansicht X in Fig. 8,

Fig. 11 ein Blockdiagramm für die Vorrichtung gemäß Fig. 1 und

Fig. 12 eine schematische Darstellung einer anderen Ausführungsform der Meßrollenabstützung.

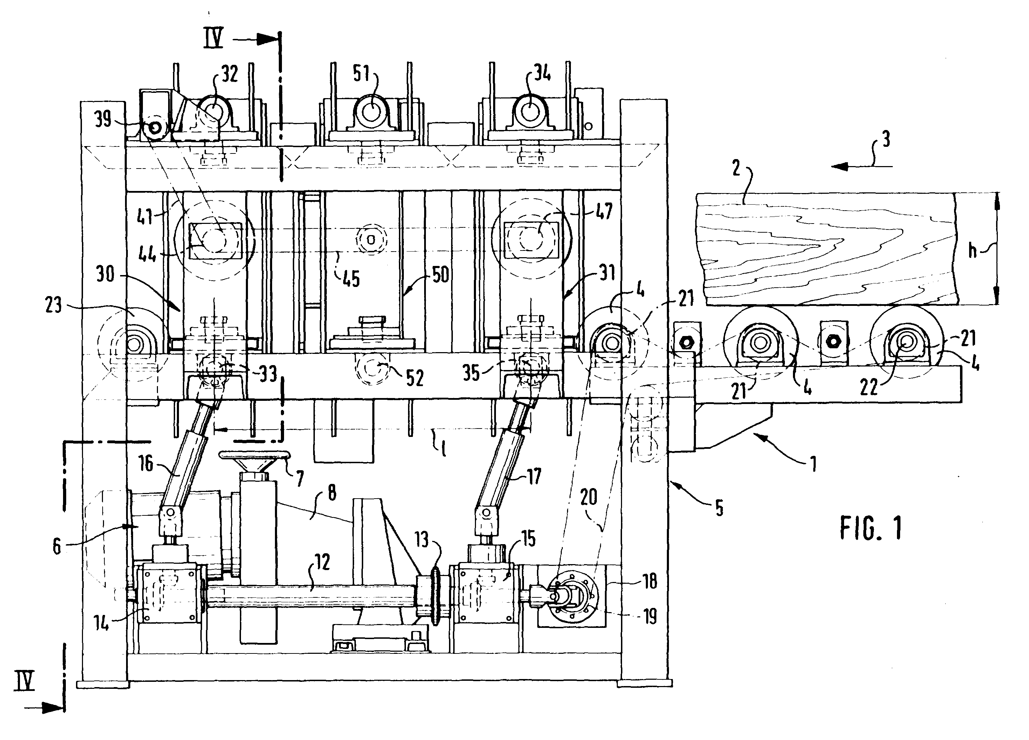

Fig. 1 zeigt eine Vorrichtung 1 zur maschinellen Festigkeitssortierung von Schnittholz 2, das hochkant in einer Bewegungsrichtung 3 auf drehend angetriebenen Rollen 4 in die Vorrichtung 1 gelangt.

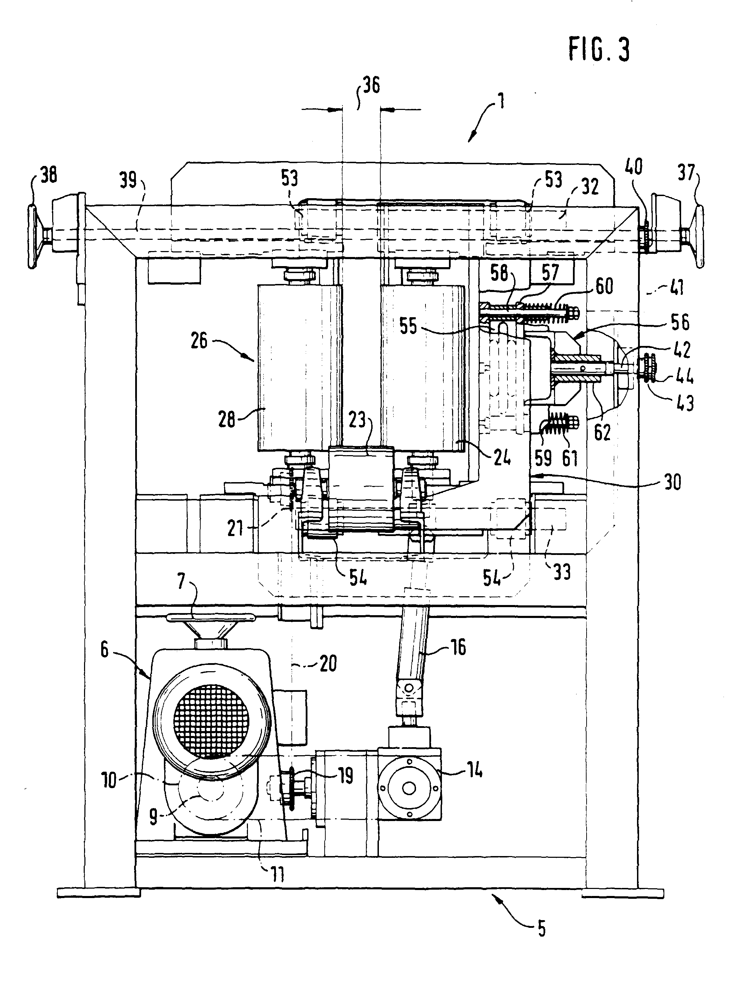

Die Vorrichung 1 weist einen stabilen Rahmen 5 auf, in dem unten ein Antrieb 6 mit einem durch ein Handrad 7 stufenlos in der Abtriebsdrehzahl einstellbaren Getriebe 8 untergebracht ist. Eine Abtriebswelle 9 (Fig. 3) des Getriebes 8 treibt ein Kettenrad 10 (Fig. 3) das über eine Kette 11 (Fig. 3) ein auf einer Welle 12 befestigtes Kettenrad 13 treibt. Die Welle 12 treibt über Kegelradgetriebe 14 und 15 teleskopierbare Gelenkwellen 16 und 17 und außerdem über einen Winkeltrieb 18 ein Kettenrad 19. Das Kettenrad 19 treibt über eine Kette 20 Kettenräder 21 auf Achsen 22 der Rollen 4.

Am Auslauf der Vorrichtung 1 befindet sich eine nicht drehend angetriebene Rolle 23 zur Stützung des auslaufenden Holzes 2.

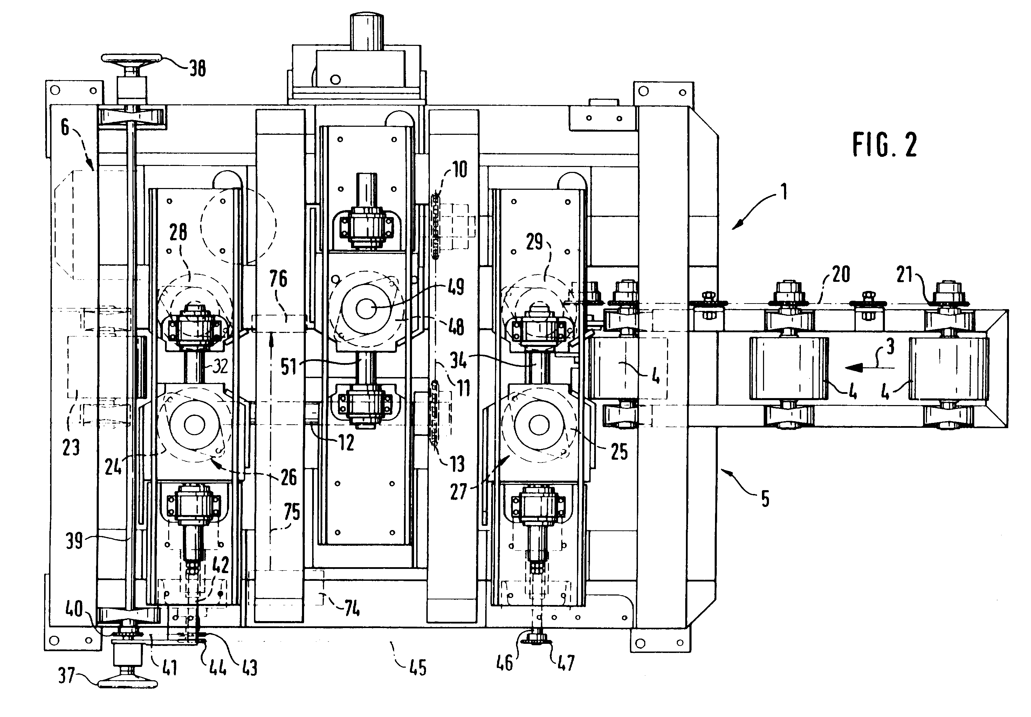

Die Gelenkwellen 16, 17 treiben jeweils eine zweite Klemmrolle 24 und 25 (Fig. 2) von Klemmrollenpaaren 26 und 27 an. Die Klemmrollenpaare 26, 27 sind in der Bewegungsrichtung 3 in einem Abstand 1, der sogenannten Stützweite, voneinander angeordnet. Jedes Klemmrollenpaar 26, 27 weist außerdem eine vorrichtungsfest drehbar gelagerte erste Klemmrolle 28 und 29 (Fig. 2) auf.

Die Längsachsen der Klemmrollen 24, 25 und 28, 29 sind sämtlich senkrecht angeordnet. Die zweiten Klemmrollen 24, 25 sind jeweils in einem Schlitten 30 und 31 drehbar gelagert. Die Schlitten 30, 31 sind jeweils auf zwei waagerechten, in senkrechtem Abstand voneinander vorrichtungsfest angeordneten Führungsstangen 32, 33 und 34, 35 rechtwinklig zu der Bewegungsrichtung 3 verschiebbar. Während in Fig. 1 die Breite h des Holzes 2 eingezeichnet ist, ist die Dicke d des Holzes 2 Fig. 4 zu entnehmen. In Abhängigkeit von der Dicke d wird der Abstand der Klemmrollen 24 und 28 sowie der Klemmrollen 25 und 29 voneinander anfänglich so eingestellt, daß dieser Ausgangsabstand 36 (Fig. 3) etwas größer als die Dicke d des Holzes 2 ist. Diese Grundeinstellung wird über ein Handrad 37 oder 38 (Fig. 2), eine an dem Rahmen 5 gelagerte Welle 39, ein darauf befestigtes Kettenrad 40 (Fig. 2), eine Kette 41, ein auf einer Spindel 42 (Fig. 2) befestigtes Kettenrad 43, ein gleiches, ebenfalls auf der Spindel 42 befestigtes Kettenrad 44, eine Kette 45 und ein wiederum gleiches, auf einer Spindel 46 befestigtes Kettenrad 47 synchron bewerkstelligt. Einzelheiten dieser Grundeinstellung werden im Zusammenhang mit Fig. 3 erläutert werden.

In der Mitte zwischen den Klemmrollenpaaren 26, 27 (Fig. 2) ist eine Meßrolle 48 frei drehbar angeordnet, deren Achse 49 parallel zu den Längsachsen der Klemmrollen 24, 25, 28, 29 ist. Die Meßrolle 48 ist an einem Schlitten 50 drehbar gelagert, der an zu den Führungsstangen 32 bis 35 parallelen, vorrichtungsfesten Führungsstangen 51 und 52 rechtwinklig zu der Bewegungsrichtung 3 verschiebbar ist.

Am Beispiel des Schlittens 30 ist in Fig. 3 gezeigt, wie der Schlitten 30 durch je zwei im Abstand voneinander angeordnete, reibungsarme Kugelbuchsen 53 und 54 auf den Führungsstangen 32, 33 verschiebbar geführt ist. Auf seiner in Fig. 3 rechten Seite stützt sich der Schlitten 30 an einem als pneumatischer Balgzylinder ausgebildeten Klemmantrieb 55 ab. Der Klemmantrieb 55 ist seinerseits rechts in Fig. 3 an einem Halter 56 abgestützt, der eine Kopfplatte 57 mit Bohrungen für Führungsbolzen 58 und 59 des Schlittens 30 aufweist. Zwischen der Kopfplatte 57 und dem freien Ende der Führungsbolzen 58, 59 ist jeweils eine Druckfeder 60 und 61 angeordnet, die den Schlitten 30 in Fig. 3 nach rechts in eine Ausgangsstellung bewegen, in der bei nicht betätigtem Klemmantrieb 55 die Klemmrollen 24, 28 in ihrem Abstand 36 voneinander angeordnet sind.

Eine Gewindebuchse 62 des Halters 56 weist ein Innengewinde auf, in das ein Außengewinde der Spindel 42 eingedreht ist. Die Spindel 42 ist in axialer Richtung festgelegt. Drehung der Spindel 42 durch den Kettentrieb 41, 43 hat daher eine axiale Verschiebung der Einheit aus Halter 56, Klemmantrieb 55 und Schlitten 30 mit zweiter Klemmrolle 24 zur Folge. Durch Drehung der Spindel 42 läßt sich auf diese Weise der den ungeklemmten Ausgangszustand charakterisierende Ausgangsabstand 36 etwas größer als die Dicke d (Fig. 4) des Holzes 2 voreinstellen.

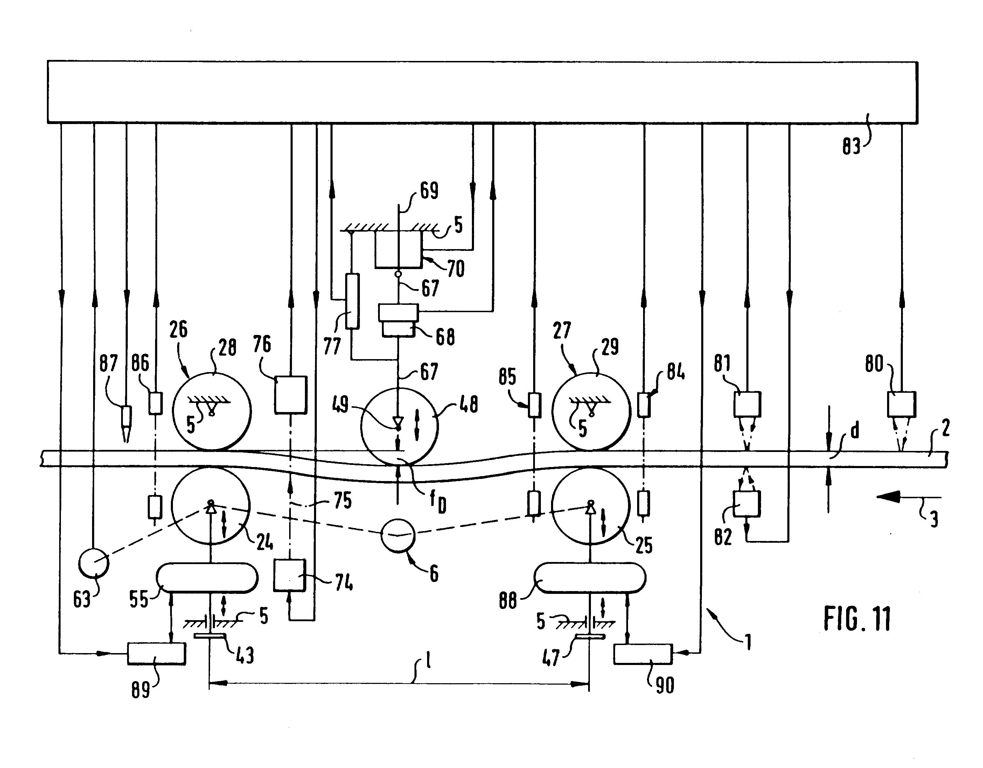

Wird dann im Prüfbetrieb, wenn sich also ein Holz 2 zwischen den Klemmwalzen 24, 28 befindet, der Klemmantrieb 55 durch Einleitung von Druckluft betätigt, bleibt der Halter 56 in seiner voreingestellten Stellung, während der Schlitten 30 in Fig. 3 nach links geschoben wird, bis die Klemmrollen 24, 28 in Klemmberührung mit dem Holz 2 getreten sind. Die Klemmkraft wird dabei so groß gewählt, daß im Prüfbetrieb zumindest annähernd kein Schlupf zwischen der drehend angetriebenen Klemmrolle 24 und dem Holz 2 auftritt. Dies ist auch deshalb von Bedeutung, weil gemäß Fig. 11 mit der Achse der Klemmrolle 24 ein Drehgeber 63 gekuppelt ist, der dem zurückgelegten Weg des Holzes 2 entsprechende elektrische Signale erzeugen soll.

Sobald das betreffende Holz 2 die Klemmrollen 24, 28 verlassen hat, wird der Klemmantrieb 55 entlüftet, so daß die Druckfedern 60, 61 den Schlitten in Fig. 3 nach rechts in seine Ausgangslage bewegen können. Die zweite Klemmrolle 24 nimmt an dieser Bewegung teil, so daß der Ausgangsabstand 36 wiederhergestellt wird. Während all dieser translatorischen Bewegungen der zweiten Klemmrolle 24 wird ihr Drehantrieb dank der Gelenkwelle 16 aufrechterhalten.

Gemäß Fig. 4 ist der Schlitten 50 mit je zwei Kugelbuchsen 64 und 65 reibungsarm auf den Führungsstangen 51, 52 verschiebbar. An den Schlitten 50 ist in Fig. 4 links an einem Anschlußpunkt 66 eine Treibstange 67 angelenkt. In die Treibstange ist eine als Kraftmeßdose ausgebildete Kraftmeßvorrichtung 68 eingesetzt. Die Kraftmeßvorrichtung 68 erzeugt elektrische Signale in Abhängigkeit von der Rückstellkraft, welche das durch die Meßrolle 48 durchgebogene Holz auf die Meßrolle 48 ausübt. Dank der soliden und reibungsarmen Führung des Schlittens 50 gelangt diese Rückstellkraft im wesentlichen vollständig und unverfälscht an die Kraftmeßvorrichtung 68.

An ihrem in Fig. 4 linken Ende ist die Treibstange 67 mit einer gegen Eigendrehung gesicherten, für axiale Verschiebung gelagerten Gewindespindel 69 eines vorrichtungsfesten Stellantriebs 70 verbunden. Mit der Gewindespindel 69 steht eine in axialer Richtung festgelegte Mutter 71 im Eingriff, die durch einen Wechselstrom-Servomotor 72 über ein Winkelgetriebe 73 drehend antreibbar ist. Eine Drehung der Mutter 71 in der einen oder anderen Richtung bewirkt also eine entsprechende translatorische Bewegung der Gewindespindel 69 und eine ebensolche Bewegung des Schlittens 50.

Fig. 4 zeigt ferner eine auch in Fig. 2 angedeutete, als Röntgenstrahler ausgebildete Strahlungsquelle 74, die einen Strahlenfächer 75 in einer zu der Bewegungsrichtung 3 des Holzes rechtwinkligen Ebene aussendet. Auf der anderen Seite des Holzes 2 ist eine als Empfängerzeile ausgebildete Empfangsvorrichtung 76 angeordnet. Die Empfangsvorrichtung 76 erzeugt elektrische Signale, die der aufgefangenen Strahlung der Strahlungsquelle 74 entsprechen, nachdem diese das Holz 2 durchdrungen hat und durch das Holz mehr oder minder absorbiert wurde. Auf diese Weise kann jeweils die gesamte Breite des Holzes 2 durch den Strahlenfächer 75 entweder kontinuierlich oder periodisch abgetastet werden. Die daraus erhaltenen elektrischen Signale können in an sich bekannter Weise ausgewertet und zu einer Beurteilung der örtlichen Ästigkeit und/oder Rohdichte des Holzes 2 herangezogen werden.

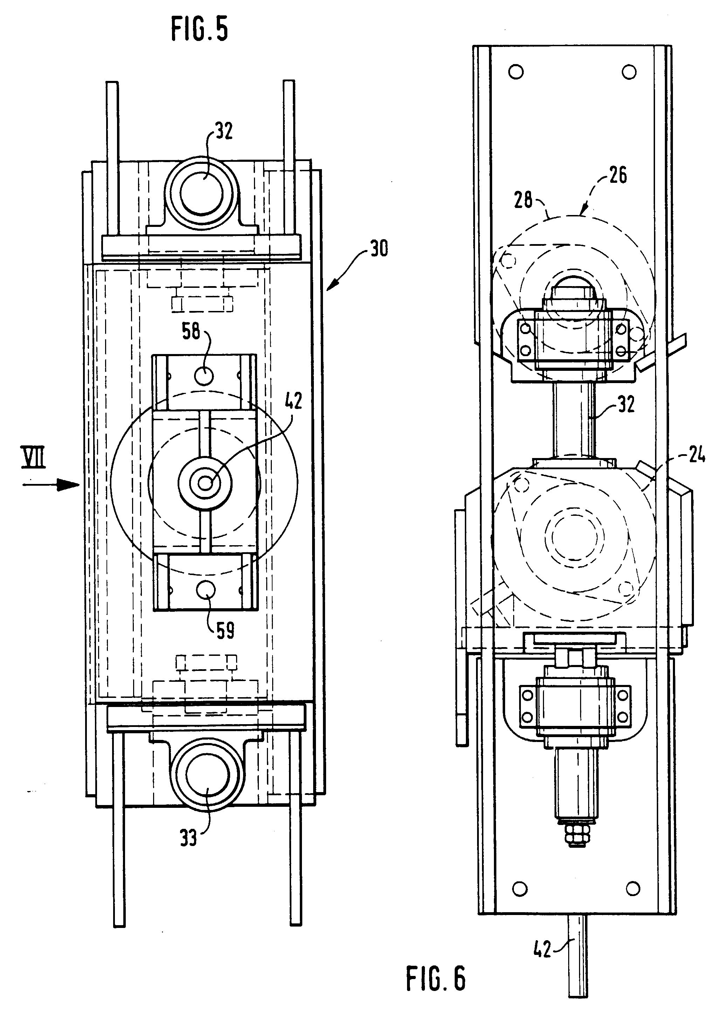

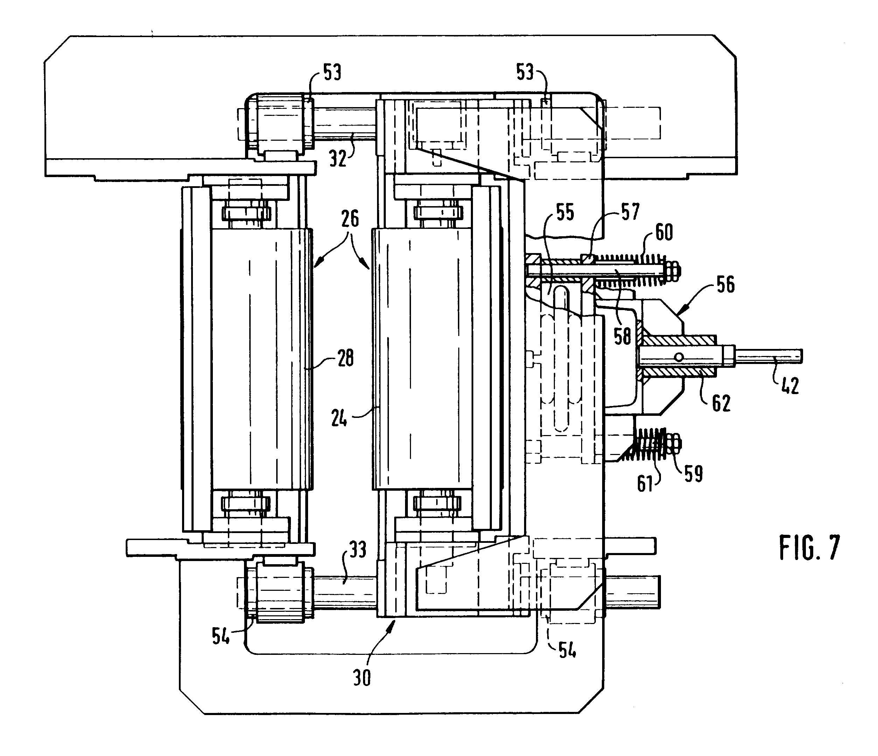

In den Fig. 5 bis 7 sind Einzelheiten des Klemmrollenpaars 26 und seiner konstruktiven Umgebung dargestellt. Die konstruktive Umgebung des anderen Klemmrollenpaars 27 ist in der gleichen Weise ausgebildet und braucht deshalb nicht ebenfalls im einzelnen gezeigt zu werden.

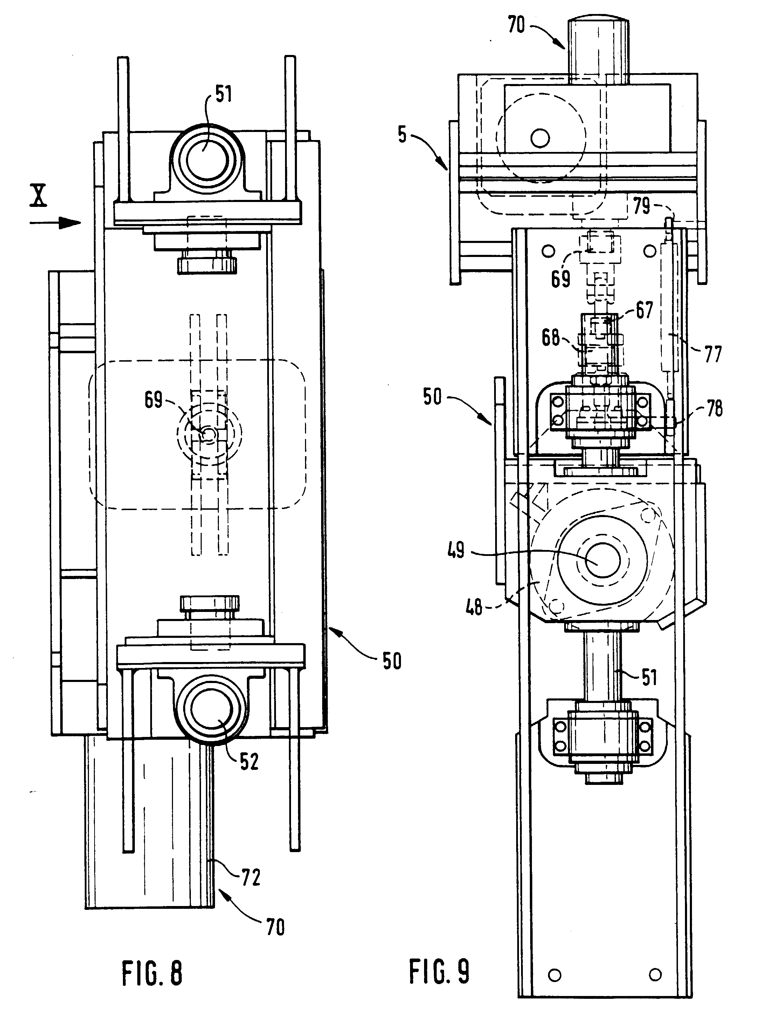

In den Fig. 8 bis 10 sind zusätzliche Details der Meßrolle 48 und der benachbarten konstruktiven Einzelheiten dargestellt.

Fig. 9 zeigt zusätzlich einen Weggeber 77 der einerseits an einen Anschlußpunkt 78 des Schlittens 50 und andererseits an einen Anschlußpunkt 79 des Rahmens 5 angelenkt ist. Der Weggeber mißt die Stellung der Meßrolle 48 rechtwinklig zu der Bewegungsrichtung 3 des Holzes 2 und erzeugt entsprechende elektrische Signale.

Das Diagramm gemäß Fig. 11 stellt in schematischer Weise Aufbau und Funktion der Vorrichtung 1 dar.

Wenn sich ein Holz 2 dem Klemmrollenpaar 27 in seiner Bewegungsrichtung 3 nähert, durchläuft es zunächst eine Meßstrecke, entlang der im Abstand voneinander Meßelemente 80 und 81 zur berührungslosen Messung der natürlichen Vorkrümmung des Holzes 2 angeordnet sind. Auf diese Weise können sehr exakt natürliche Vorkrümmungen festgestellt werden, die das Holz 2 in Fig. 11 entweder nach oben oder nach unten von der dort gezeichneten neutralen Mittellage aufweist. Als Meßelemente kommen insbesondere Lasersensoren in Betracht.

Dem Meßelement 81 liegt auf der anderen Seite des Holzes 2 ein Meßelement 82 gegenüber. Die Meßköpfe 81, 82 dienen dazu, die Dicke d des Holzes 2 zu messen. Alle Meßelemente 80 bis 82 sind mit einem Rechner 83 verbunden und liefern elektrische Meßsignale in den Rechner 83.

Der Anfang und das Ende des Holzes 2 betätigen nacheinander Lichtschranken 84, 85 und 86 der Vorrichtung 1. Auch diese Lichtschranken sind zur Signalübermittlung mit dem Rechner 83 verbunden.

Desgleichen sind die Strahlungsquelle 74 zur Steuerung und die Empfangsvorrichtung 76 zur Signalübermittlung mit dem Rechner 83 verbunden.

Durch den Rechner 83 kann ferner eine Einrichtung 87 zum Markieren des Holzes 2 gesteuert werden. Der durch die zweite Klemmrolle 24 drehend antreibbare inkrementale Drehgeber 63 speist seine Signale ebenfalls in den Rechner 83 ein. Gleiches gilt für die Kraftmeßvorrichtung 68 und den Weggeber 77.

Der Klemmantrieb 55 für die zweite Klemmrolle 24 und ein gleichartiger Klemmantrieb 88 für die zweite Klemmrolle 25 werden jeweils durch ein pneumatisches Wegeventil 89 und 90 betätigt. Die Wegeventile 89, 90 werden durch den Rechner 83 gesteuert.

Die Vorrichtung 1 funktioniert beispielsweise wie folgt:

Durch die Vorderkante des Holzes 2 wird die Lichtschranke 84 unterbrochen, während die sich noch in der Offenstellung befindenden zweiten Klemmrolle 24, 25 schon drehend angetrieben werden. Das hat zur Folge, daß der Drehgeber 63 inkrementale Signale in den Rechner 83 eingibt. Nach einer bestimmten Anzahl dieser Inkremente befindet sich bei bekannter Vorschubgeschwindigkeit des Holzes 2 der Anfang des Holzes schon kurz hinter dem Klemmrollenpaar 27. Dann steuert der Rechner das Wegeventil 90 an, und der Klemmantrieb 88 wird mit Druckluft beaufschlagt. Das hat zur Folge, daß die zweite Klemmrolle 25 in Berührung mit dem Holz 2 geklemmt wird und von da ab an dem Vorschub des Holzes 2 in seiner Bewegungsrichtung 3 teilnimmt. Die Vorderkante des Holzes durchbricht sodann die Lichtschranke 85. Dadurch wird die Messung der natürlichen Vorkrümmung des Holzes 2 durch die Meßelemente 80, 81 und die Messung der Dicke d des Holzes 2 begonnen.

Der Anfang des Holzes 2 trifft sodann auf die Meßrolle 48 und wird von dieser in Fig. 11 nach unten hin abgelenkt. Der Anfang des Holzes 2 durchläuft dann den Strahlenfächer 75, so daß sofort durch die Empfangsvorrichtung 76 Signale entsprechend der Ästigkeit und Rohdichte des Holzes 2 in den Rechner 83 eingegeben werden können. Der Anfang des Holzes 2 durchdringt dann das Klemmrollenpaar 26, dessen zweite Klemmrolle 24 sich noch in der Offenstellung befindet. Kurz darauf wird nach einer entsprechenden Anzahl Inkremente das Wegeventil 89 durch den Rechner 83 angesteuert und beaufschlagt den Klemmantrieb 55 mit Druckluft. Das hat zur Folge, daß die zweite Klemmrolle 24 in Klemmberührung mit dem Holz 2 gefahren wird. Kurz darauf unterbricht der Anfang des Holzes 2 die Lichtschranke 86, wodurch die Kraftmessung der Kraftmeßvorrichtung 68 und die Wegmessung des Weggebers 77 begonnen werden. Aufgrund der Voreinstellung der Meßrolle 48 durch die Gewindespindel 69 ergibt sich an dem Holz 2 in der Ebene der Meßrolle 48 eine erzwungene Durchbiegung $f_D$. Ziel ist es, diese erzwungene Durchbiegung $f_D$ zumindest annähernd konstant zu halten. Dies wird durch Regelung des Stellantriebs 70 durch den Rechner 83 unter Berücksichtigung der durch die Meßelemente 80, 81 festgestellten natürlichen Vorkrümmung des Holzes 2 bewerkstelligt. Dabei wird die Ist-Position der Meßrolle 48 quer zur Bewegungsrichtung 3 des Holzes 2 ständig durch den Weggeber 77 an den Rechner 83 übermittelt.

Die Größe der erzwungenen Durchbiegung $f_D$ wird in Abhängigkeit vor allem von der Dicke d des Holzes 2 so gewählt, daß das Holz 2 nicht zu gering, aber auch nicht überbeansprucht wird.

Aus der Rückstellkraft aufgrund der erzwungenen Durchbiegung $f_D$ wird in gewissen Abständen, z.B. alle 10 mm Holzlänge, der Biege-Elastizitätsmodul durch den Rechner 83 errechnet. Diese Information faßt der Rechner 83 mit den aus der Durchstrahlung mit dem Strahlenfächer 75 über die gesamte Holzlänge gewonnenen Festigkeitsinformation zusammen und gewinnt einen kombinierten Festigkeitswert, den er mit den Grenzen zuvor einprogrammierter Sortierklassen vergleicht. Das Ergebnis kann in geeigneter Weise durch die Markiereinrichtung 87 auf dem Holz 2 markiert werden, um die nachfolgende Sortierung nach Sortierklassen, gegebenenfalls nach vorherigem Kappen an Qualitätsgrenzen, zu erleichtern.

Der Durchlauf des Endes des Holzes 2 durch die Vorrichtung 1 hat nacheinander die Beendigung der zuvor aktivierten Funktionen zur Folge.

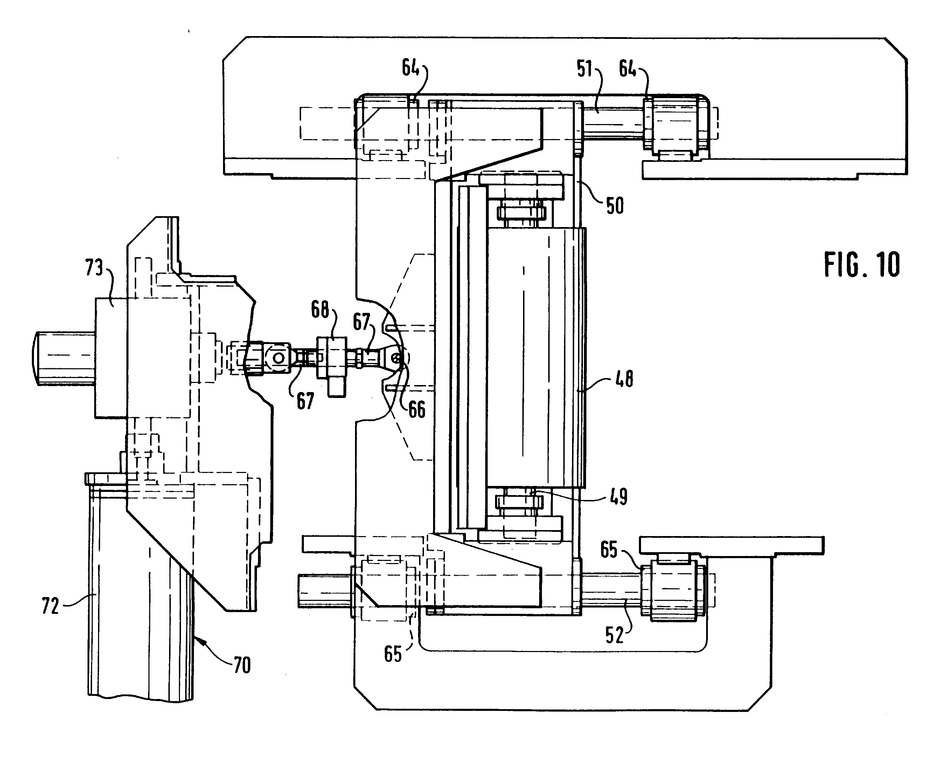

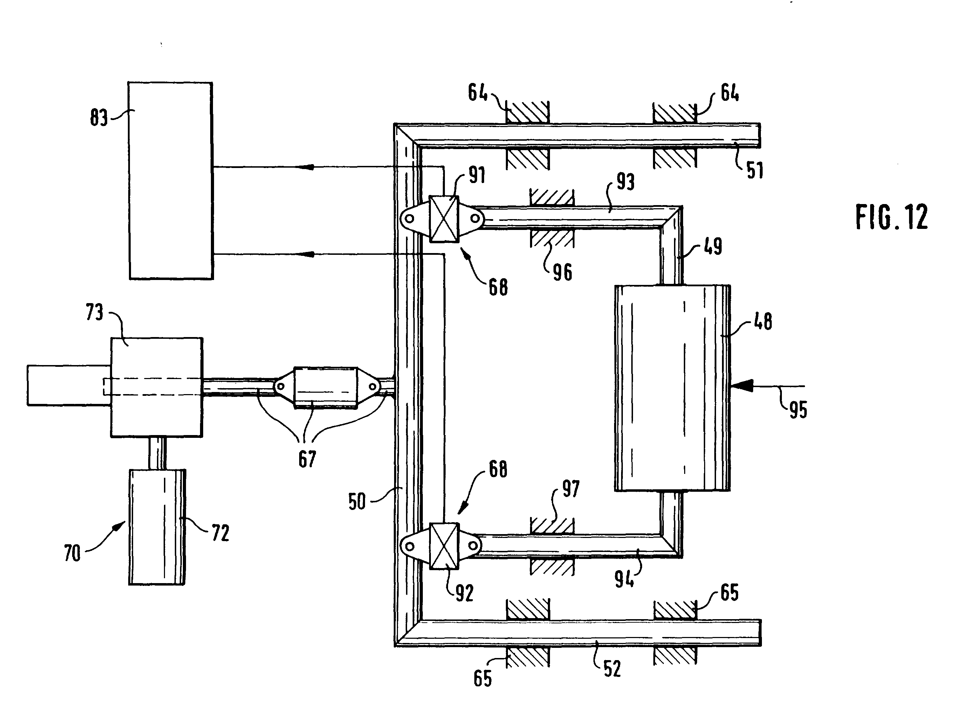

Fig. 12 zeigt eine andere Ausführungsform der Abstützung der Achse 49 der Meßrolle 48. Die Kraftmeßvorrichtung ist in Fig. 12 nicht, wie bei Fig. 10, in die Treibstange 67 integriert. Die Kraftmeßvorrichtung 68 gemäß Fig. 12 weist vielmehr zwei Kraftmeßelemente 91 und 92 auf, die jeweils einerseits an dem Schlitten 50 und andererseits an einer Führungsstange 93 und 94 angelenkt sind. Jede Führungsstange 93, 94 ist an einem Ende der Achse 49 befestigt.

Durch einen Pfeil 95 ist die resultierende Kraft angedeutet, die durch das in Fig. 12 nicht gezeichnete Holz auf die Meßrolle 48 ausgeübt wird. Die resultierende Kraft 95 greift in Fig. 12 in der Längsmitte der Meßrolle 48 an. Selbst wenn dies bei sich ändernden Abmessungen des Holzes nicht mehr der Fall sein sollte, die resultierende Kraft also jenseits der Längsmitte der Rolle 48 angreifen würde, wäre eine einwandfreie Kraftmessung gewährleistet, weil Verkantungen einerseits in den Kugelbuchsen 64,65 der Führungsstangen 51, 52 und andererseits in Kugelbuchsen 96 und 97 der Führungsstangen 93, 94 ausgeschlossen sind.

In der Ausführungsform nach Fig. 12 weisen gleiche Teile wie in der vorangehend beschriebenen Ausführungsform gleiche Bezugszahlen auf.

**Patentansprüche**

1. Verfahren zur maschinellen Festigkeitssortierung von Schnittholz,

bei dem das Holz (2) durch zwei im Abstand voneinander angeordnete Klemmrollenpaare (26,27) gestützt wird,

und bei dem eine Meßrolle (48) zwischen den Klemmrollenpaaren (26,27) mit dem Holz (2) zusammenwirkt, quer zu einer Bewegungsrichtung (3) des Holzes (2) bewegbar ist und eine Durchbiegung ($f_D$) des Holzes (2) erzwingt,

wobei die aufgrund der Durchbiegung ($f_D$) des Holzes (2) auf die Meßrolle (48) ausgeübte Rückstellkraft des Holzes (2) periodisch gemessen, in Form entsprechender Meßsignale in einen Rechner (83) eingegeben und zur Abschätzung der örtlichen Festigkeit des Holzes (2) durch den Rechner (83) verwendet wird,

dadurch gekennzeichnet, daß die natürliche Vorkrümmung des Holzes (2) gemessen und entsprechende Meßsignale in den Rechner (83) eingegeben werden,

daß die Meßrolle (48) während des Meßbetriebs durch einen Stellantrieb (70) in ständige Berührung mit dem Holz (2) gedrückt wird,

daß die Stellung der Meßrolle (48) quer zu der Bewegungsrichtung (3) des Holzes (2) gemessen und entsprechende Meßsignale in den Rechner (83) eingegeben werden,

und daß der Stellantrieb (70) durch den Rechner (83) so geregelt wird, daß im Meßbetrieb die durch die Meßrolle (48) erzwungene Durchbiegung ($f_D$) des Holzes (2) zumindest annähernd konstant ist.

2. Verfahren nach Anspruch 1, dadurch gekennzeichnet, daß die Größe der erzwungenen Durchbiegung ($f_D$) in Abhängigkeit von dem zu messenden Holz (2) so gewählt wird, daß das Holz (2) nicht zu gering, aber auch nicht überbeansprucht wird.

3. Verfahren nach Anspruch 1 oder 2, dadurch gekennzeichnet, daß der Betriebspunkt der Stellung der Meßrolle (48) quer zu der Bewegungsrichtung (3) des Holzes (2) zumindest annähernd in dem optimalen Bereich der Spannungs/Dehnungskurve des betreffenden Holzes (2) gehalten wird.

4. Verfahren nach einem der Ansprüche 1 bis 3, dadurch gekennzeichnet, daß die Klemmrollen (24,28;25,29) jedes Klemmrollenpaars (26;27) auf einen Ausgangsabstand (36) voneinander eingestellt werden, der wenigstens gleich der jeweiligen Holzdicke (d) ist,
und daß ein Klemmantrieb (55;88) jedes Klemmrollenpaars (26;27) erst dann zur Klemmung des Holzes (2) eingeschaltet wird, wenn die Vorderkante des Holzes (2) das betreffende Klemmrollenpaar (26;27) passiert hat.

5. Verfahren nach Anspruch 4, dadurch gekennzeichnet, daß der Ausgangsabstand (36) der Klemmrollen (24,28; 25,29) jedes Klemmrollenpaars (26;27) wiederhergestellt wird, sobald die Hinterkante des Holzes (2) das Klemmrollenpaar (26;27) verlassen hat.

6. Verfahren nach einem der Ansprüche 1 bis 5, dadurch gekennzeichnet, daß die natürliche Vorkrümmung des Holzes (2) berührungslos gemessen (80,81) wird.

7. Verfahren nach einem der Ansprüche 1 bis 6, dadurch gekennzeichnet, daß die Holzdicke (d) gemessen (81, 82) und entsprechende Meßsignale in den Rechner (83) eingegeben werden.

8. Verfahren nach einem der Ansprüche 1 bis 7, dadurch gekennzeichnet, daß mit dem Rechner (83) periodisch der örtliche Biege-Elastizitätsmodul (E) des Holzes (2) nach folgender Formel errechnet wird:

$$E = \frac{F \cdot l^3}{48 \cdot (f_D \pm f_N) \cdot \dfrac{h \cdot d^3}{12}} \ [N/mm^2]$$

worin

F = die jeweilige Rückstellkraft,

l = der Abstand zwischen den Klemmrollenpaaren (26,27),

$f_D$ = die durch das Drücken mit der Meßrolle (48) erzwungene Durchbiegung des Holzes (2),

$f_N$ = die natürliche Vorkrümmung des Holzes (2),

h = die in den Rechner (83) eingegebene Breite des Holzes (2)
und

d = die jeweilige Holzdicke

sind.

9. Verfahren nach einem der Ansprüche 1 bis 8, dadurch gekennzeichnet, daß das Holz (2) örtlich durchstrahlt (74,75) wird, und daß Strahlen nach dem Verlassen des Holzes (2) aufgefangen (76), in elektrische Signale umgewandelt und die Signale zur Bestimmung der örtlichen Ästigkeit und/oder der örtlichen Rohdichte dem Rechner (83) zugeführt werden.

10. Verfahren nach Anspruch 9, dadurch gekennzeichnet, daß das Holz (2) mit Röntgenstrahlen (75) durchstrahlt wird und Röntgenstrahlen nach dem Verlassen des Holzes (2) durch eine quer zu der Bewegungsrichtung (3) des Holzes (2) angeordnete Zeile (76) von Empfängern aufgefangen werden.

11. Verfahren nach Anspruch 9 oder 10, dadurch gekennzeichnet, daß der örtliche Biege-Elastizitätsmodul (E) sowie die örtliche Ästigkeit und/oder die örtliche Rohdichte des Holzes (2) rechnerisch zusammengefaßt und gemeinsam zur Ermittlung der örtlichen Festigkeit des Holzes (2) herangezogen werden.

12. Verfahren nach einem der Ansprüche 1 bis 11, dadurch gekennzeichnet, daß die minimale örtliche Festigkeit eines Holzes (2) durch den Rechner (83) ermittelt und mit durch Bereiche von Festigkeitswerten festgelegten Sortierklassen verglichen wird und danach eine Sortierung des Holzes (2) in die zutreffende Sortierklasse erfolgt.

13. Verfahren nach einem der Ansprüche 1 bis 11, dadurch gekennzeichnet, daß bei auftretenden Schwankungen in der örtlichen Festigkeit eines Holzes (2) zugehörige Qualitäts- bzw. Sortierklassen und deren Grenzen an dem Holz (2) festgestellt und später zum Kappen dieses Holzes (2) und damit zur Teilung des Holzes (2) in Teilhölzer unterschiedlicher Sortierklassen herangezogen werden.

14. Vorrichtung (1) zur maschinellen Festigkeitssortierung von Schnittholz,

mit zwei im Abstand (1) voneinander angeordneten Klemmrollenpaaren (26,27) zur Stützung und zum Vorschub des Holzes (2) in eine Bewegungsrichtung (3),

mit einer zwischen den Klemmrollenpaaren (26,27) angeordneten, mit dem Holz (2) zusammenwirkenden, eine Durchbiegung ($f_D$) des Holzes (2) erzwingenden, drehbar gelagerten Meßrolle (48), und

mit einer Kraftmeßvorrichtung (68),

wobei eine Achse (49) der Meßrolle (48) quer zu der Bewegungsrichtung (3) des Holzes (2) bewegbar und an der Kraftmeßvorrichtung (68) abgestützt ist,

und wobei der durch das Holz (2) auf die Meßrolle (48) ausgeübten Rückstellkraft entsprechende Meßsignale der Kraftmeßvorrichtung (68) in einen Rechner (83) eingebbar sind,

dadurch gekennzeichnet, daß eine Einrichtung (80,81) zur Messung der natürlichen Vorkrümmung des Holzes (2) vorgesehen ist, durch die entsprechende Meßsignale in den Rechner (83) eingebbar sind,

daß ein Stellantrieb (70) vorgesehen ist,

daß die Meßrolle (48) während des Meßbetriebs durch den Stellantrieb (70) in ständige Berührung mit dem Holz (2) drückbar ist,

daß eine Einrichtung (77) zur Messung der Stellung der Meßrolle (48) quer zu der Bewegungsrichtung (3) des Holzes (2) vorgesehen ist, durch die entsprechende Meßsignale in den Rechner (83) eingebbar sind,

und daß der Stellantrieb (70) durch den Rechner (83) so regelbar ist, daß im Meßbetrieb die durch die Meßrolle (48) erzwungene Durchbiegung ($f_D$) des Holzes (2) zumindest annähernd konstant ist.

15. Vorrichtung nach Anspruch 14, dadurch gekennzeichnet, daß von jedem Klemmrollenpaar (26;27) eine erste Klemmrolle (28;29) vorrichtungsfest drehbar gelagert ist, eine zweite Klemmrolle (24;25) an einem quer zu der Bewegungsrichtung (3) des Holzes (2) verschiebbaren Schlitten (30;31) drehbar gelagert ist, und wenigstens eine (24;25) der Klemmrollen (24,28;25,29) drehend antreibbar ist, und daß jeder Schlitten (30;31) durch einen steuerbaren (89;90) Klemmantrieb (55;88) verschiebbar ist.

16. Vorrichtung nach Anspruch 15, dadurch gekennzeichnet, daß jeder Klemmantrieb (55;88) an einem Halter (56) abgestützt ist, und daß jeder Halter (56) in Abhängigkeit von der jeweiligen Holzdicke (d) in der Verschieberichtung (3) des zugehörigen Schlittens (30;31) voreinstellbar (42,62) ist.

17. Vorrichtung nach Anspruch 16, dadurch gekennzeichnet, daß zwischen jedem Schlitten (30;31) und dem zugehörigen Halter (56) eine Federanordnung (60,61) vorgesehen ist, durch welche der Schlitten (30;31) in eine Öffnungsrichtung weg von der ersten Klemmrolle (28; 29) vorgespannt ist.

18. Vorrichtung nach einem der Ansprüche 14 bis 17, dadurch gekennzeichnet, daß die natürliche Vorkrümmung des Holzes (2) durch wenigstens zwei jeweils im Abstand voneinander in der Bewegungsrichtung (3) des Holzes (2) vor dem zuerst mit dem Holz in Berührung tretenden Klemmrollenpaar (27) angeordnete Meßelemente (80,81) berührungslos meßbar ist, und daß jedes Meßelement (80,81) elektrische Meßsignale erzeugt und mit dem Rechner (83) verbunden ist.

19. Vorrichtung nach einem der Ansprüche 14 bis 18, dadurch gekennzeichnet, daß die Holzdicke (d) durch zwei in größerem Abstand als die Holzdicke (d) voneinander quer zu der Bewegungsrichtung (3) des Holzes (2) angeordnete Meßelemente (81,82) berührungslos meßbar ist, und daß jedes Meßelement (81;82) elektrische Meßsignale erzeugt und mit dem Rechner (83) verbunden ist.

20. Vorrichtung nach einem der Ansprüche 14 bis 19, dadurch gekennzeichnet, daß eine das Holz (2) quer zu seiner Bewegungsrichtung (3) durchstrahlende (75) Strahlungsquelle (74) vorgesehen ist,

daß auf der von der Strahlungsquelle (74) abgewandten Seite des Holzes (2) eine Empfangsvorrichtung (76) für durch das Holz (2) hindurchgedrungene Strahlen angeordnet ist,

und daß die Empfangsvorrichtung (76) der aufgefangenen Strahlung entsprechende elektrische Signale erzeugt und mit dem Rechner (83) verbunden ist.

21. Vorrichtung nach Anspruch 20, dadurch gekennzeichnet, daß die Strahlungsquelle (74) als Röntgenstrahler ausgebildet ist, der einen Strahlenfächer (75) in einer zu der Bewegungsrichtung (3) des Holzes (2) zumindest annähernd rechtwinkligen Ebene aussendet, und daß die Empfangsvorrichtung (76) eine in dieser Ebene angeordnete Empfängerzeile aufweist.

22. Vorrichtung nach Anspruch 21, dadurch gekennzeichnet, daß die Empfängerzeile (76) die gesamte Breite (h) des Holzes (2) abtastet.

23. Vorrichtung nach einem der Ansprüche 14 bis 21, dadurch gekennzeichnet, daß die Meßrolle (48) an einem quer zu der Bewegungsrichtung (3) des Holzes (2) verschiebbaren Schlitten (50) drehbar gelagert ist, und daß eine die Kraftmeßvorrichtung (68) aufweisende Treibstange (67) an dem Schlitten (50) angelenkt und durch den vorrichtungsfesten Stellantrieb (70) verschiebbar ist.

24. Vorrichtung nach einem der Ansprüche 14 bis 21, dadurch gekennzeichnet, daß der vorrichtungsfeste Stellantrieb (70) über eine Treibstange (67) mit einem quer zu der Bewegungsrichtung (3) des Holzes (2) verschiebbaren Schlitten (50) verbunden ist, und daß beide Enden der Achse (49) der Meßrolle (48) über je ein Kraftmeßelement der Kraftmeßvorrichtung (63) an dem Schlitten (50) abgestützt sind.

25. Vorrichtung nach Anspruch 23 oder 24, dadurch gekennzeichnet, daß der Stellantrieb (70) einen durch den Rechner (83) zur Einhaltung einer zumindest annähernd konstanten, durch die Meßrolle (48) erzwungenen Durchbiegung ($f_D$) des Holzes (2) regelbaren Wechselstrom-Servomotor (72) aufweist.

26. Vorrichtung nach einem der Ansprüche 23 bis 25, dadurch gekennzeichnet, daß die Einrichtung zur Messung der Stellung der Meßrolle (48) einen einerseits (78) an dem Schlitten (50) und andererseits (79) vorrichtungsfest (5) abgestützten Weggeber (77) aufweist.

27. Vorrichtung nach einem der Ansprüche 14 bis 26, dadurch gekennzeichnet, daß an einem Auslaß der Vorrichtung (1) eine durch den Rechner (83) steuerbare Einrichtung (87) zum Markieren des durchgelaufenen Holzes (2) angeordnet ist.

**Claims**

1. Process for the machine sorting of sawn timber according to strength,

   in which the timber (2) is supported by two pairs of clamping rollers (26, 27) arranged spaced from each other,

   and in which a test roller (48) cooperates with the timber (2) between the clamping roller pairs (26, 27).is movable transversely to a direction of movement (3) of the timber (2), and imposes a bending deflection ($f_D$) on the timber (2),

   wherein the restoring force of the timber (2) exerted on the test roller (48) as a result of the bending deflection ($f_D$) of the timber (2) is periodically measured, and corresponding measurement signals are generated and fed to a computer (83) and are used for the calculation of the local strength of the timber (2) by the computer (83).

   characterised in that the natural curvature of the timber (2) is measured and corresponding measurement signals are fed to the computer (83),

   the test roller (48) is pressed into continuous contact with the timber (2) during the measurement process by a setting drive (70),

   the position of the test roller (48) transversely to the direction of movement (3) of the timber (2) is measured and corresponding measurement signals are fed to the computer (83).

   and the setting drive (70) is so controlled by the computer (83) that in the measurement process the bending deflection ($f_D$) of the timber (2) imposed by the test roller (48) is at least approximately constant.

2. Process according to claim 1, characterised in that the magnitude of the imposed flexible bending ($f_D$) is chosen in dependence on the timber (2) under test so that the timber (2) is not stressed too little but also is not overstressed.

3. Process according to claim 1 or 2, characterised in that the working point for the setting of the test roller (48) transversely to the direction of movement (3) of the timber (2) is maintained at least approximately in the optimum region of the stress/extension curve of the timber (2).

4. Process according to one of claims 1 to 3, characterised in that the clamping rollers (24, 28; 25, 29) of each clamping roller pair (26; 27) are adjusted to an initial gap (36) from one another which is at least equal to the particular timber thickness (d), and a clamping drive (55; 88) of each clamping roller pair (26; 27) is first actuated for the clamping of the timber (2) when the leading edge of the timber (2) has passed the relevant clamping roller pair (26; 27).

5. Process according to claim 4, characterised in that the initial gap (36) of the clamping rollers (24, 28; 25, 29) of each clamping roller pair (26; 27) is reproduced as soon as the trailing edge of the timber (2) has left the clamping roller pair (26; 27).

6. Process according to one of claims 1 to 5, characterised in that the natural curvature of the timber (2) is measured (80, 81) without contact.

7. Process according to one of claims 1 to 6, characterised in that the timber thickness (d) is measured (81, 82) and corresponding measurement signals are fed to the computer (83).

8. Process according to one of claims 1 to 7, characterised in that the computer (83) periodically calculates the local modulus of elasticity in flexure (E) of the timber (2) according to the following equation:

$$E = \frac{F \cdot l^3}{48 \cdot (f_D \pm f_N) \cdot \frac{h \cdot d^3}{12}} \ [N/mm^2]$$

wherein

F = the particular restoring force
l = the span between the clamping roller pairs (26, 27)
$f_D$ = the bending deflection of the timber (2) imposed by the pressure of the test roller (48)
$f_N$ = the natural curvature of the timber (2)
h = the width of the timber (2) entered into the computer (83) and
d = the particular thickness of the timber.

9. Process according to one of claims 1 to 8, characterised in that the timber (2) is locally irradiated (74, 75), and radiation detected (76) after passing through the timber (2) is converted into electrical signals and the signals are fed to the computer (83) for determining the local knottiness and/or the local gross density.

10. Process according to claim 9, characterised in that the timber (2) is irradiated by X-rays (75) and X-rays after passing through the timber (2) are detected by a line (76) of receivers arranged transversely to the direction of movement (3) of the timber (2).

11. Process according to claim 9 or 10, characterised in that the local modulus of elasticity in flexure (E) as well as the local knottiness and/or the local gross density of the timber (2) are combined together by computation and are used jointly for the establishment of the local strength of the timber (2).

12. Process according to one of claims 1 to 11, characterised in that the minimum local strength of a piece of timber (2) is determined by the computer (83) and is compared with sorting classes established by bands of strength values, and thereafter the timber (2) is sorted into the relevant sorting classes.

13. Process according to one of claims 1 to 11, characterised in that on the occurrence of variations in the local strength of a piece of timber (2) appropriate qualitative or sorting classes and their limits are identified for the timber (2) and subsequently are used for the cutting-up of this timber (2) and the dividing of the timber (2) into timber parts of different sorting classes.

14. Apparatus (1) for the machine sorting of sawn timber according to strength,

comprising two pairs (26, 27) of clamping rollers arranged at a spacing (1) from one another for supporting and advancing the timber (2) in a direction of movement (3).

a rotatably mounted test roller (48) which cooperates with the timber (2), imposes a bending deflection ($f_D$) on the timber (2) and is arranged between the clamping roller pairs (26, 27),

and a force measuring device (68),

wherein a shaft (49) of the test roller (48) is movable transversely to the direction of movement (3) of the timber (2) and is supported by the force measuring device (68).

and wherein measurement signals from the force measuring device (68) corresponding to the restoring force exerted by the timber (2) on the test roller (48) are fed to a computer (83),

characterised in that there is provided means (80, 81) for measuring the natural curvature of the timber (2) and from which corresponding measurement signals are arranged to be fed to the computer (83),

there is provided a setting drive (70).

the test roller (48) is arranged to be pressed into continuous contact with the timber (2) during the measurement process by the setting drive (70).

means (77) are provided for measuring the position of the test roller (48) transversely to the direction of movement (3) of the timber (2) and from which the appropriate measurement signals are arranged to be fed to the computer (83),

and the setting drive (70) is controllable by the computer (83) so that in the measurement process the bending deflection ($f_D$) of the timber (2) imposed by the test roller (48) is at least approximately constant.

15. Apparatus according to claim 14, characterised in that of each clamping roller pair (26; 27) a first clamping roller (28; 29) is rotatably mounted fixedly on the apparatus, a second clamping roller (24; 25) is rotatably mounted on slide means (30; 31) displaceable transversely to the direction of movement (3) of the timber (2), and at least one (24; 25) of the clamping rollers (24, 28; 25, 29) is rotatably drivable,
and each slide means (30; 31) is displaceable by a controllable (89; 90) clamping drive (55; 88).

16. Apparatus according to claim 15, characterised in that each clamping drive (55; 88) is supported by a holder (56), and each holder (56) is pre-settable (42, 62) in the direction of displacement (3) of the associated slide means (30; 31) in dependence upon the particular timber thickness (d).

17. Apparatus according to claim 16, characterised in that between each slide means (30; 31) and the associated holder (56) there is provided spring means (60, 61) by which the slide means (30; 31) is urged in a direction of opening away from the first clamping roller (28; 29).

18. Apparatus according to one of claims 14 to 17, characterised in that the natural curvature of the timber (2) is arranged to be measured without contact by at least two measuring elements (80, 81) which are arranged spaced from one another in the direction of movement (3) of the timber (2) in advance of the clamping roller pair (27) which first comes into contact with the timber, and each measuring element (80; 81) produces electrical measurement signals and is connected to the computer (83).

19. Apparatus according to one of claims 14 to 18, characterised in that the timber thickness (d) is arranged to be measured without contact by two measuring elements (81, 82) which are arranged at a greater distance apart than the timber thickness (d) transversely to the direction of movement (3) of the timber (2), and each measuring element (81; 82) produces electrical measurement signals and is connected to the computer (83).

20. Apparatus according to one of claims 14 to 19, characterised in that a radiation source (74) is provided which is arranged to irradiate (75) the timber (2) transversely to its direction of movement (3),

receiving means (76) for radiation which has passed through the timber (2) is arranged on the side of the timber (2) which is remote from the radiation source (74),

and the receiving means (76) produces electrical signals corresponding to the detected radiation and is connected to the computer (83).

21. Apparatus according to claim 20, characterised in that the radiation source (74) is an X-ray source which emits a fan of radiation (75) in a plane which is at least approximately perpendicular to the direction of movement (3) of the timber (2), and the receiving means (76) comprises a line of receivers arranged in this plane.

22. Apparatus according to claim 21, characterised in that the line of receivers (76) senses the full width (h) of the timber (2).

23. Apparatus according to one of claims 14 to 21, characterised in that the test roller (48) is rotatably mounted on a slide carriage (50) which is displaceable transversely to the direction of movement (3) of the timber (2), and a connecting rod (67) incorporating the force measuring device (68) is coupled to the slide carriage (50) and is displaceable by the setting drive (70) which is fixed in position on the apparatus.

24. Apparatus according to one of claims 14 to 21, characterised in that the setting drive (70) which is fixed in position on the apparatus is connected by means of a connecting rod (67) to a slide carriage (50) which is displaceable transversely to the direction of movement (3) of the timber (2), and the two ends of the shaft (49) of the test roller (48) are respectively supported on the slide carriage (50) by respective force measuring elements of the force measuring device (68).

25. Apparatus according to claim 23 or 24, characterised in that the setting drive (70) comprises an ac servo-motor (72) which is controllable by the computer (83) in order to maintain an at least approximately constant bending ($f_D$) of the timber (2) imposed by the test roller (48).

26. Apparatus according to one of claims 23 to 25 characterised in that the means for measuring the position of the test roller (48) comprises a displacement transducer (77) which is mounted on the one hand (78) on the slide carriage (50) and on the other hand (79) fixedly (5) on the apparatus.

27. Apparatus according to one of claims 14 to 26, characterised in that at an exit from the apparatus (1) there is provided means (87) controllable by the computer (83) for marking the passed timber (2).

## Revendications

1. Procédé de tri mécanique du bois de coupe en fonction de sa solidité,

dans lequel le bois (2) est soutenu par deux paires de galets de serrage (26, 27) disposés à distance l'une de l'autre,
et dans lequel un rouleau de mesure (48) agit sur le bois (2) entre les paires de galets de serrage (26, 27), est mobile perpendiculairement à un sens de déplacement (3) du bois (2) et impose une déflexion ($f_D$) au bois (2),
la force de rappel du bois (2) exercée sur le rouleau de mesure (48) par la déflexion ($f_D$) du bois (2) étant mesurée périodiquement, transmise à un ordinateur (83) sous la forme de signaux de mesure appropriés et utilisée par l'ordinateur (83) pour estimer la solidité locale du bois (2),

caractérisé en ce que la flexion naturelle préexistante du bois (2) est mesurée et les signaux de mesure correspondants sont transmis à l'ordinateur (83),

en ce que le rouleau de mesure (48) est appuyé en contact permanent avec le bois (2) pendant la mesure par un vérin (70),
en ce que la position du rouleau de mesure (48) perpendiculairement au sens de déplacement (3) du bois (2) est mesurée et les signaux de mesure correspondants sont transmis à l'ordinateur (83),
et en ce que le vérin (70) est contrôlé par l'ordinateur (83) de telle sorte que la déflexion ($f_D$) imposée au bois (2) par le rouleau de mesure (48) pendant la mesure soit au moins approximativement constante.

2. Procédé selon la revendication 1, caractérisé en ce que la grandeur de la déflexion ($f_D$) imposée est sélectionnée en fonction du bois (2) à mesurer de telle sorte que la contrainte exercée sur le bois (2) ne soit pas trop faible mais pas non plus excessive.

3. Procédé selon la revendication 1 ou 2, caractérisé en ce que le point d'action dans la position du rouleau de mesure (48) perpendiculairement au sens de déplacement (3) du bois (2) est maintenu au moins approximativement dans la plage optimale de la courbe de tension/dilatation du bois (2) concerné.

4. Procédé selon l'une ou l'ensemble des revendications 1 à 3, caractérisé en ce que les galets de serrage (24, 28 ; 25, 29) de chaque paire de galets de serrage (26 ; 27) sont placés l'un par rapport à l'autre à une distance de départ (36) au moins égale à l'épaisseur (d) du bois,

et en ce qu'un entraînement de serrage (55 ; 88) de chaque paire de galets de serrage (26 ; 27) n'est actionné pour serrer le bois (2) que lorsque le bord antérieur du bois (2) a franchi la paire de galets de serrage (26 ; 27) concernée.

5. Procédé selon la revendication 4, caractérisé en ce que la distance de départ (36) des galets de serrage (24, 28 ; 25, 29) de chaque paire de galets de serrage (26 ; 27) est rétablie dès que le bord postérieur du bois (2) est sorti de la paire de galets de serrage (26 ; 27).

6. Procédé selon l'une ou l'ensemble des revendications 1 à 5, caractérisé en ce que la flexion naturelle préexistante du bois (2) est mesurée (80, 81) sans contact.

7. Procédé selon l'une ou l'ensemble des revendications 1 à 6, caractérisé en ce que l'épaisseur du bois (d) est mesurée (81, 82) et les signaux de mesure correspondants sont transmis à l'ordinateur (83).

8. Procédé selon l'une ou l'ensemble des revendications 1 à 7, caractérisé en ce que l'ordinateur (83) calcule périodiquement le module d'élasticité en flexion (E) local du bois (2) selon la formule suivante :

$$E = \frac{F \times l^3}{48 \times (f_D \pm f_N) \times \dfrac{h \times d^3}{12}} \; [\text{N/mm}^2]$$

où :

F =   force de rappel

l =   distance entre les paires de galets de serrage (26 ; 27)

$f_D$ =   déflexion imposée au bois (2) par la pression du rouleau de mesure (48)

$f_N$ =   flexion naturelle préexistante du bois (2)

h =   largeur du bois (2) entrée dans l'ordinateur (83) et

d =   épaisseur du bois

9.   Procédé selon l'une ou l'ensemble des revendications 1 à 8, caractérisé en ce que le bois (2) est localement exposé à un rayonnement (74, 75) et le rayonnement est capté (76) après qu'il sort du bois (2), converti en signaux électriques, et les signaux sont transmis à l'ordinateur (83) afin de déterminer la densité de noeuds locale et/ou la densité apparente locale.

10.   Procédé selon la revendication 9, caractérisé en ce que le bois (2) est exposé à des rayons X (75) et des rayons X sont captés après leur sortie du bois (2) par une rangée de récepteurs (76) disposée perpendiculairement au sens de déplacement (3) du bois (2).

11.   Procédé selon la revendication 9 ou 10, caractérisé en ce que le module d'élasticité en flexion local (E) et la densité de noeuds locale et/ou la densité apparente locale du bois (2) sont réunis par le calcul et utilisés ensemble pour déterminer la solidité locale du bois (2).

12.   Procédé selon l'une ou l'ensemble des revendications 1 à 11, caractérisé en ce que la solidité minimale locale d'un bois (2) est déterminée par l'ordinateur (83) et comparée avec des catégories de tri déterminées par des plages de valeurs de solidité, et le bois (2) est ensuite classé dans la catégorie de tri correspondante.

13.   Procédé selon l'une ou l'ensemble des revendications 1 à 11, caractérisé en ce qu'en cas de fluctuations de la solidité locale d'un bois (2), les catégories de qualité ou de tri correspondantes et leurs limites sont déterminées sur le bois (2) et prises en compte par la suite pour l'éboutage de ce bois (2) et pour partager ainsi le bois (2) en pièces correspondant aux différentes catégories de tri.

14.   Dispositif (1) de tri mécanique du bois de coupe en fonction de sa solidité, comportant

deux paires de galets de serrage (26 ; 27) disposées à distance (1) l'une de l'autre pour soutenir et faire avancer le bois (2) dans un sens d'avancement (3),
un rouleau de mesure (48) supporté avec possibilité de rotation entre les paires de galets de serrage (26 ; 27), agissant sur le bois et imposant au bois (2) une déflexion ($f_D$), et un dispositif dynamométrique (68),
dans lequel un axe (49) du rouleau de mesure (48) est mobile perpendiculairement au sens de déplacement (3) du bois (2) et s'appuie sur le dispositif dynamométrique (68),
et dans lequel des signaux de mesure du dispositif dynamométrique (68) correspondant à la force de rappel exercée par le bois (2) sur le rouleau de mesure (48) peuvent être transmis à un ordinateur (83),

caractérisé en ce qu'il est prévu un dispositif (80, 81) pour la mesure de la courbure naturelle préexistante du bois (2) qui peut transmettre les signaux de mesure correspondants à l'ordinateur (83),

en ce qu'il est prévu un vérin (70),
en ce que le rouleau de mesure (48) est appuyé en contact permanent avec le bois (2) pendant la mesure par le vérin (70),
en ce qu'il est prévu un dispositif (77) pour mesurer la position du rouleau de mesure (48) perpendiculairement au sens de déplacement (3) du bois (2), qui peut transmettre les signaux de mesure correspondants à l'ordinateur (83),
et en ce que le vérin (70) est contrôlé par l'ordinateur (83) de telle sorte que la déflexion ($f_D$) imposée au bois (2) par le rouleau de mesure (48) pendant la mesure soit au moins approximativement constante.

15.   Dispositif selon la revendication 14, caractérisé en ce que dans chaque paire de galets de serrage (26 ; 27), un premier galet de serrage (28 ; 29) est supporté avec possibilité de rotation de manière solidaire du dispositif, un deuxième galet de serrage (24 ; 25) est supporté avec possibilité de rotation sur un chariot (30 ; 31) coulissant perpendiculairement au sens de déplacement (3) du bois (2), et au moins un (24 ; 25) des galets de serrage (24, 28 ; 25, 29) peut être entraîné en rotation,
et en ce que chaque chariot (30 ; 31) peut être déplacé par un entraînement de serrage (55 ; 88) pouvant être commandé (89 ; 90).

16.   Dispositif selon la revendication 15, caractérisé en ce que chaque entraînement de serrage (55; 88) est appuyé sur un support (56), et en ce que chaque support (56) peut être ajusté au préalable en fonction de l'épaisseur (d) du bois dans le sens de déplacement (3) du chariot (30 ; 31) correspondant.

17.   Dispositif selon la revendication 16, caractérisé en ce qu'il est prévu entre chaque chariot (30 ; 31) et le support (56) correspondant un dispositif de ressort (60, 61) qui précontraint le chariot (30 ; 31) dans une position d'ouverture écartée du premier galet de serrage (28 ; 29).

18. Dispositif selon l'une ou l'ensemble des revendications 14 à 17, caractérisé en ce que la courbure naturelle du bois (2) peut être mesurée sans contact par au moins deux éléments de mesure (80, 81) disposés à distance l'un de l'autre dans le sens de déplacement (3) du bois (2) devant la paire de galets de serrage (27) qui vient la première en contact avec le bois, et en ce que chaque élément de mesure (80 ; 81) génère des signaux électriques et est relié à l'ordinateur (83).

19. Dispositif selon l'une ou l'ensemble des revendications 14 à 18, caractérisé en ce que l'épaisseur du bois (d) peut être mesurée sans contact par deux éléments de mesure (81 ; 82) disposés l'un par rapport à l'autre à une distance plus grande que l'épaisseur (d) du bois perpendiculairement au sens de déplacement (3) du bois (2), et en ce que chaque élément de mesure (80 ; 81) génère des signaux électriques et est relié à l'ordinateur (83).

20. Dispositif selon l'une ou l'ensemble des revendications 14 à 18, caractérisé en ce qu'il est prévu une source de rayonnement (74) émettant un rayonnement (75) vers le bois (2) perpendiculairement au sens de déplacement (3) de celui-ci,

en ce qu'un dispositif récepteur (76) destiné au rayonnement traversant le bois (2) est disposé sur le côté du bois (2) opposé à la source de rayonnement (74),
et en ce que le dispositif récepteur (76) génère des signaux électriques correspondant au rayonnement reçu et est relié à l'ordinateur (83).

21. Dispositif selon la revendication 20, caractérisé en ce que la source de rayonnement (74) est conçue comme un émetteur de rayons X, qui projette un faisceau de rayons en éventail (75) dans un plan au moins approximativement perpendiculaire au sens de déplacement (3) du bois (2), et en ce que le dispositif récepteur (76) présente une rangée de récepteurs disposée dans ce même plan.

22. Dispositif selon la revendication 21, caractérisé en ce que la rangée de récepteurs (76) assure la détection sur toute la largeur (h) du bois (2).

23. Dispositif selon l'une ou l'ensemble des revendications 14 à 21, caractérisé en ce que le rouleau de mesure (48) est supporté avec possibilité de rotation sur un chariot (50) coulissant perpendiculairement au sens de déplacement (3) du bois (2), et en ce qu'une tringle (67) portant le dispositif dynamométrique (68) est articulée sur le chariot (50) et peut être déplacée par le vérin (70) fixé au dispositif.

24. Dispositif selon l'une ou l'ensemble des revendications 14 à 21, caractérisé en ce que le vérin (70) fixé sur le dispositif est relié par une tringle (67) à un chariot (50) coulissant perpendiculairement au sens de déplacement (3) du bois (2), et en ce que les deux extrémités de l'axe (49) du rouleau de mesure (48) s'appuient chacune sur le chariot (50) par l'intermédiaire d'un élément dynamométrique du dispositif dynamométrique (68).

25. Dispositif selon la revendication 23 ou 24, caractérisé en ce que le vérin (70) présente un servomoteur à courant alternatif (72) pouvant être réglé par l'ordinateur (83) en vue de maintenir au moins approximativement constante la déflexion($f_D$) imposée au bois (2) par le rouleau de mesure (48).

26. Dispositif selon l'une ou l'ensemble des revendications 23 à 25, caractérisé en ce que le dispositif de mesure de la position du rouleau de mesure (48) présente un capteur de déplacement (77) appuyé d'un côté (78) sur le chariot (50) et solidaire du dispositif (5) de l'autre côté (79).

27. Dispositif selon l'une ou l'ensemble des revendications 14 à 26, caractérisé en ce qu'un dispositif (87) contrôlable destiné à marquer le bois (2) qui le franchit est disposé à une sortie du dispositif (1).

FIG. 1

FIG. 2

# FIG. 3

**FIG. 4**

FIG.5

32

30

VII

58

42

59

33

26

28

32

24

42

FIG. 6

FIG. 7

FIG. 8

FIG. 9

FIG. 10

EP 0 632 886 B1

FIG. 11

23

FIG.12